# EUROPÄISCHE PATENTSCHRIFT

(11) **EP 2 008 667 B1**
(45) Veröffentlichungstag und Bekanntmachung des Hinweises auf die Patenterteilung: **16.10.2013**
(21) Anmeldenummer: 08011032.3
(22) Anmeldetag: 18.06.2008
(51) Int. Cl.: A61L 2/06, B29C 49/66, B29C 35/16

(54) **Vorrichtung zum Behandeln von Gefäßen in einem Gefäßdesinfektionssystem**
Device for treating containers in a container disinfection system
Dispositif de traitement de récipient dans un système de désinfection des récipients

(30) Priorität: 18.06.2007 DE 102007028471
(43) Veröffentlichungstag der Anmeldung: 31.12.2008
(73) Patentinhaber: Krones AG, 93073 Neutraubling (DE)
(72) Erfinder: Burgmeier, Berthold, 89561 Dischingen/Eglingen (DE); Engelhard, Patrik, 84094 Elsendorf (DE)

(56) Entgegenhaltungen:
- EP-A- 0 303 135
- DE-A1- 2 605 967
- DE-A1-102006 036 763
- DE-A1-102006 053 193
- DE-B- 1 133 082
- US-A- 2 501 193
- US-A1- 2004 208 781

## Beschreibung

Die Erfindung betrifft ein Verfahren zum Behandeln von Gefäßen in einem Gefäßdesinfektionssystem gemäß dem Oberbegriff des Anspruchs 1.

Es ist bekannt, offene Gefäße aus Kunststoff nach dem Herstellprozess noch vor dem Befüllen mit einem Produkt, das durch Einwirkung von Keimen, Hefen, Sporen oder Organismen jeglicher Art verderben kann, durch eine Desinfektionsbehandlung, bevorzugt auf chemischem Wege, insbesondere mittels H₂O₂ so zu behandeln, dass sie vor dem Einbringen des Produkts, insbesondere ein Getränk oder sonstiges Lebensmittel, in einer Füllmaschine in einem für den weiteren Prozess unbedenklichen Zustand zur Verfügung gestellt werden (siehe beispielsweise EP 1 012 047 B1). Für die Behandlung des Gefäßinnenraums mit einem Desinfektionsmittel, vorzugsweise gasförmigem Wasserstoffperoxyd (H₂O₂), ist eine gleichmäßige Oberflächentemperatur im Gefäßinnenraum von Vorteil. Bei der Herstellung von Kunststoffflaschen, beispielsweise durch einen Streck-Blas-Prozess in einer Streckblasmaschine besteht jedoch die Problematik, dass die Flaschen beim Verlassen der Streckblasmaechine auf Grund verschiedener Umstände, z.B. der Kühlmittelführung in den Blasformen, der unterschiedlichen Materialverteilung bezogen auf den Flaschenlängsquerschnitt sowie der Strömung der Streckblasluft im Innenraum einer Kunststoffflaache Zonen mit zum Teil erheblich unterschiedlichen Oberflächentemperaturen aufweisen. Dies ist in Bezug auf eine möglichst gleichmäßige Sterilisationsbehandlung von Nachteil. So kann an kühleren Flächenbereichen eines Gefäßes eine Kondensation des Sterilisationsmittels auftreten, während an heißeren Bereichen die Dampfphase erhalten bleibt.

Aus der US2004208781 ist ein Verfahren zum Behandeln von Gefäßen in einem Gefäßdesinfektionssystem bekannt, bei dem die Gefäße von einer Gefäßherstelleinrichtung zu einer Sterilisationseinrichtung überführt werden, wobei während dem Überführen ein aktiver Wärmeausgleich im Innenraum der Gefäße durch Einleiten eines Fluids erfolgt.

Die DE2605967 offenbart eine Blasformmaschine durch welche Kunststoffbehälter hergestellt werden, Die Behälter werden dabei bereits in der Blasform durch Einsprühen von Nebel gekühlt, sodass die spätere Abkühlzeit verringert werden kann.

Aus der US 2 501 193 ist ein Flaschensterilisator für Babyflaschen bekannt, der einen tauschbaren Dorn aufweist, der in die Flasche eindringt, wenn diese mit geöffneter Mündung in den Sterilisator gestellt wird. Durch den Dorn wird das Sterilisationsmittel in die nähe des Bodens gebracht.

Die DE 11 33 082 zeigt eine Sterilisationseinrichtung für Flaschen, wobei diese zur Außenumgebung hin abgeschlossen sind und mittels Dampf unter Druck ausgeblasen werden. Die Einrichtung weist ferner einstellbare Überströmventile auf, um den Dampfdruck in der zu sterilisierenden Flasche einstellen zu können

DE 10 2006 053193 zeigt eine Vorrichtung zum Herstellen von Kunststoffflaschen, bei der die Flaschen nach der Herstellung in einen Kühltunnel gefahren werden, um von außen gekühlt zu werden. Die Kühlung erfolgt zur Einstellung der Temperatur vor weiteren Behandlungsschritten.

Eine Leiteinrichtung für Sterilisationsmedium in Form einer glockenförmigen Hülse zeigt die DE 10 2006 036 763. Damit soll in eine Flasche eingeleitetes Sterilisationsmittel beim Austritt nach der Sterilisation umgelenkt werden, um auch Außenbereiche der Flaschen behandeln zu können.

Demgegenüber liegt der Erfindung die Aufgabe zu Grunde, eine Verbesserung zur Sicherstellung einer gleichmäßigen, von der Lage der Gefäßoberflächenbereiche unabhängigen Sterilisationsbehandlung sicherzustellen.

Gelöst wird diese Aufgabe durch die kennzeichnenden Merkmale des Anspruchs 1.

Dies geschieht durch einen vor der eigentlichen Sterilisationsbehandlung erfolgenden aktiven Temperaturausgleich im Innenraum eines Gefäßes, vorzugsweise einem aus Kunststoff hergestellten Gefäß, insbesondere einer PET-Flasche für Getränke oder dgl., wobei bevorzugt ein gasförmiges Fluid, wie z.B. Sterilluft mit einer vorbestimmten Temperatur in den Gefäßinnenraum eingeleitet wird, die mit einer unter der Temperatur der heißesten Stelle des unmittelbar zuvor hergestellten Gefäßes liegenden Eigentemperatur eingeleitet wird. Die Fluidführung der erzeugten Strömung ist so ausgestaltet, dass das durch die offene Mündung eingeleitete Fluid zunächst auf die Zone mit der höchsten Oberflächentemperatur des Gefäßinnenraums geleitet wird, um von diesem Bereich einen Teil der Wärmeenergie durch Abkühlen der Gefäßoberfläche aufzunehmen und im weiteren Strömungsverlauf anderen Gefäßoberflächenzonen zuzuführen, die nach dem Herstellprozess eine zunächst geringere Temperatur aufweisen. Im Idealfall wird durch die Fluidströmung ein annähernd gleichmäßiges Temperaturprofil über die gesamte Höhe der Gefäßinnenfläche durch Wärmeausgleich erreicht, bevor das in ein Gefäß eingeleitete Fluid den Gefäßinnenraum durch die offene Mündung wieder verlässt.

Da bei der Herstellung von Flaschen, insbesondere für Getränke oder dgl., das Bodenzentrum mit dem Anspritzpunkt in der Regel den Bereich höchster Temperatur darstellt, ist es besonders zweckmäßig, das zum Temperaturausgleich verwendete Fluid, vorzugsweise Sterilluft, durch die offene Gefäßmündung zunächst senkrecht auf diesen zentralen, die höchste Oberflächentemperatur aufweisenden Bodenbereich zu richten, wo das eine geringere Temperatur aufweisende gasförmige Fluid Wärme aufnimmt und im weiteren Verlauf durch Umlenkung an der Bodenfläche und nachfolgender Aufwärtsströmung entlang der Seitenwände über die Schulterfläche bis zur Mündung Wärme an Oberflächenabschnitte mit geringerer Temperatur abgibt, so dass insgesamt gesehen ein annähernd gleichmäßiger Temperaturausgleich über die gesamte Flaschenhöhe erfolgt, bevor das Sterilisationsmittel, vorzugsweise Wasserstoffperoxid, eingeleitet wird.

Dies kann bevorzugt durch eine zumindest teilweise durch die Flaschenmündung in den Innenraum hineinreichende Kanüle bewerkstelligt werden, die annähernd koaxial ausgerichtet die Flaschenmündung zumindest teilweise in axialer Richtung durchgreift. Oberhalb der offenen Flaschenmündung kann bevorzugt an der genannten Kanüle ein annähernd schirm- bzw. haubenartiger, die Flaschenmündung und wenigstens einen Teil der Mündungsaußenwand umfassender Umlenkkörper angeordnet sein, der die aus dem freien Ringquerschnitt zwischen der Kanüle und der Flaschenmündung axial nach oben austretende Luft um annähernd 180 Grad umlenkt und zumindest teilweise um die bevorzugt ein Schraubgewinde aufweisende äußere Mündungsseitenfläche herumführt, um diesen im Regelfall zu den kühleren Flaschenzonen gehörenden Bereich durch Warmluftkontakt zusätzlich auch von außen zu erwärmen.

Bevorzugt wird Sterilluft in einem Temperaturbereich von 50 bis 80 Grad Celsius, insbesondere von 50 bis 60 Grad Celsius in den Flascheninnenraum eingeleitet. Die im Einzelfall jeweils ideale Lufttemperatur hängt von verschiedenen Parametern, wie Material- und Temperaturverteilung innerhalb einer frisch geblasenen Flasche, deren Volumen, der Behandlungszeit und dem Luftvolumenstrom sowie anderen Faktoren ab. Idealerweise wird die Lufttemperatur so gewählt, dass die nach dem Temperaturausgleich vorliegende Oberflächentemperatur noch über dem Taupunkt des verwendeten Sterilisationsmittels liegt.

Die aktive Temperaturausgleichsbehandlung und die nachfolgende Sterilisationsbehandlung sind nicht nur zeitlich versetzt, sondern erfolgen bevorzugt auch räumlich getrennt, beispielsweise in verschiedenen Sternrädern oder Karussells, wodurch mit der Trennung eine Verschleppung von Sterilisationsmitteln verhinderbar ist.

Nachfolgend wird ein Bbeispiel anhand der Figur erläutert.

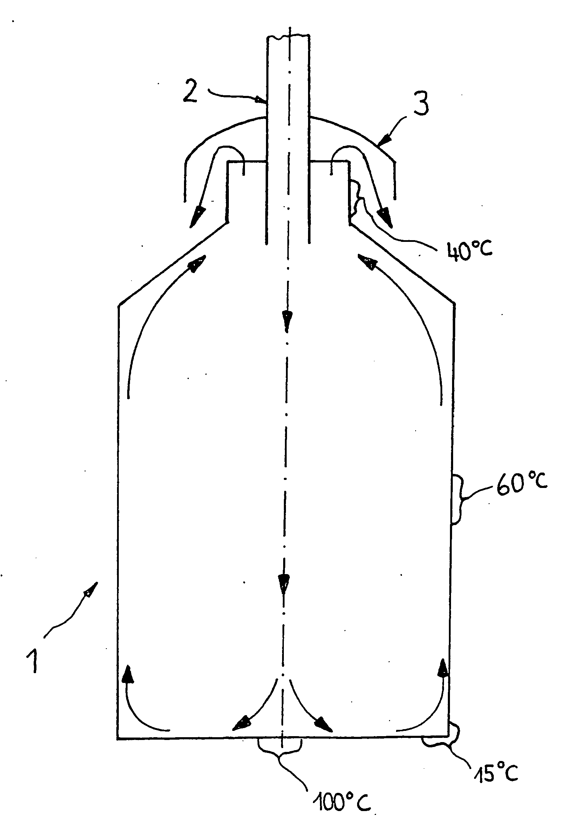

In einer Vorrichtung zum Herstellen, Sterilisieren, Füllen und Verschließen von Kunststoffflaschen, wie besonders anschaulich aus der Figur 1 der eingangs genannten EP 1 012 047 B1 bekannt, befindet sich zwischen einer Streckblasmaschine und einer in Flaschenflussrichtung nachgelagerten Sterilisationsmaschine eine Transferstrecke, die in der Regel aus einem oder mehreren Sternrädern gebildet wird, wobei die soeben aus einem erhitzten Vorformling geblasenen und noch heißen Flaschen unter Beibehaltung eines Teilungsabstandes weitergeleitet werden. Die über ihre Höhe ein zunächst noch ungleichmäßiges Innenflächentemperaturprofil aufweisenden Flaschen werden auf der Transferstrecke zum Sterilisator einer aktiven Wärmeausgleichsbehandlung mit einem gasförmigen Fluid, vorzugsweise Sterilluft, unterzogen, das in den offenen Innenraum eingeleitet wird.

Dazu wird im Bereich dieser Wegstrecke mit jeder offenen Flasche 1 zumindest zeitweise eine in der Figur dargestellte Kanüle 2 synchron mitgeführt, die annähernd koaxial zur Flaschenachse ausgerichtet die Mündung durchgreift und teilweise in den Flascheninnenraum hinein ragt.

An dieser Kanüle 2 befindet sich ein mit Abstand oberhalb zum Mündungsrand angebrachter haubenartig ausgebildeter Umlenkkörper 3, der die Mündungsaußenseitenwand, an der sich in der Regel ein Schraubgewinde befindet, zumindest teilweise mit einem freien Radialspalt umfänglich umgibt.

Das gemeinsame Gebilde aus Kanüle und Umlenkkörper ist in nicht näher gezeigter weise höhenbeweglich und kann beispielsweise über eine Kurvensteuerung eine bestimmte Wegstrecke mit der bevorzugt kontinuierlich transportierten Flasche 1 mitgeführt, oder über steuerbare Betätigungselemente wie Pneumatikzylinder oder dgl. unabhängig von wechselnden Laufgeschwindigkeiten zeitkonstant in der gezeigten Stellung in Eingriff gehalten werden. Dieser Vorgang kann beispielsweise in einem dafür speziell eingerichteten Sternrad stattfinden.

Durch die Kanüle wird erwärmte Sterilluft mit einer Temperatur im Bereich zwischen 50 - 80 Grad Celsius, vorzugsweise aber zwischen 50 - 60 Grad in den Flascheninnenraum axial von oben mit einem Volumenstrom von 5 - 15 Normkubikmetern pro Stunde einströmend in Richtung auf die heißeste, annähernd 100 Grad Celsius aufweisende Stelle im Bodenzentrum mit dem Anspritzpunkt geleitet. Beim Auftreffen der kühleren Sterilluft auf diesen Zentralbereich wird diese radial zur Seite umgelenkt und nimmt dabei Wärme auf, d.h. das Bodenzentrum wird abgekühlt. Die erwärmte Luft gibt dagegen in dem nur ca. 15 Grad kühlen Übergangsbereich vom Boden zur senkrechten Flaschenseitenwand Wärme ab und strömt anschließend an der rund 60 Grad warmen Wandfläche nach oben über die schräg nach innen geneigten Schulterfläche zum ungefähr 40 Grad warmen Mündungsbereich, wo sie zwischen der Kanüle und der Mündungsinnenseitenwand durch den freien Ringquerschnitt aus der Flaschenmündung axial nach oben austritt, dabei auf den Umlenkkörper trifft und von diesem um ca. 180° um die Flaschenmündung herumgeführt und annähernd senkrecht nach unten gerichtet wird. Dabei streicht die warme Luft auch an der äußeren Mündungsseitenfläche vorbei.

Noch vor dem Erreichen des Sterilisators wird die Kanüle nach oben über den Mündungsrand der Flasche angehoben und im weiteren Verlauf einer nachfolgenden Flasche zugeführt. Ein Transfersternrad kann an seinem Umfang mit einer Vielzahl solcher bevorzugt heb- und senkbaren Kanülen ausgestattet sein. Erst nach dem aktiven Temperaturausgleich wird der Innenraum einer in den Sterilisator überführten Flasche mit Wasserstoffperoxid oder dgl. beaufschlagt.

Die Erfindung ist aber nicht auf die Anwendung in Sternrädern beschränkt, sondern kann in angepasster Weise auch in Verbindung mit geradlinigen Transfereinrichtungen, wie Zugmittelstrecken (Ketten, Zahnriemen) oder dgl. zum Einsatz kommen.

Der sich ergebende Vorteil der vorgeschlagenen Verfahrensweise ist primär darin zu sehen, dass aufgrund gleichmäßiger Temperaturverhältnisse an der Gefäßwandung auch gleiche Rahmenbedingungen für die Sterilisationsbehandlung gegeben sind und beispielsweise Kondensatbildung von Wasserstoffperoxid oder dgl. vermieden werden kann.

## Patentansprüche

1. Verfahren zum Behandeln von Gefäßen in einem Gefäßdesinfektionssystem bei dem die Gefäße von einer Gefäßherstelleinrichtung, insbesondere einer Streckblasmaschine für Kunststoffgefäße, zu einer Sterilisationseinrichtung überführt werden, wobei während dem Überführen ein aktiver Wärmeausgleich im Innenraum der Gefäße durch Einleiten eines Fluids, insbesondere Sterilluft, erfolgt,
**dadurch gekennzeichnet, dass**
das Fluid mit einer Temperatur in das Gefäß eingeleitet wird, die unter der Temperatur einer heißesten Zone des Gefäßes liegt, wobei das Fluid zunächst auf die heißeste Zone geleitet wird.

2. Verfahren nach Anspruch 1, **dadurch gekennzeichnet, dass** das Fluid beim Eintritt in den Innenraum eines Gefäßes eine Temperatur aufweist, die höher als die kälteste Zone im Gefäß ist.

3. Verfahren nach wenigstens einem der Ansprüche 1 oder 2, **dadurch gekennzeichnet, dass** die Temperatur des eingeführten Fluids so gewählt ist, dass die Innenfläche eines Gefäßes nach dem Temperaturausgleich eine über dem Taupunkt des Sterilisationsmittels liegende Temperatur aufweist.

## Claims

1. Method for treating containers in a container sterilisation system in which the containers are transferred from a container production device, in particular a stretch blow-forming machine for plastics containers, to a sterilisation device wherein an active temperature equalisation takes place in the interior of the containers during the transfer through the introduction of a fluid, in particular sterile air,
**characterised in that**
the fluid is introduced into the container at a temperature below the temperature of a hottest zone of the container, the fluid being directed first at the hottest zone.

2. Method according to Claim 1, **characterised in that** upon entering the interior of a container the fluid has a temperature which is higher than the coldest zone in the container.

3. Method according to at least one of Claims 1 and 2, **characterised in that** the temperature of the fluid introduced is selected such that the internal surface of a container has a temperature above the dew point of the sterilisation medium after the temperature equalisation.

## Revendications

1. Procédé de traitement de récipients dans un système de désinfection de récipients selon lequel les récipients sont transférés d'un dispositif de fabrication, en particulier d'une machine d'étirage-gonflage pour le formage de récipients en matière plastique vers un dispositif de stérilisation, pendant le transfert une égalisation active de la température à la partie interne des récipients étant effectuée par introduction d'un fluide, en particulier d'air stérile,
**caractérisé en ce que**
le fluide est introduit dans le récipient à une température située au-dessous de la température de la zone la plus chaude de ce récipient, ce fluide étant tout d'abord transféré sur la zone la plus chaude.

2. Procédé conforme à la revendication 1,
**caractérisé en ce qu'**
à son entrée dans le volume interne d'un récipient le fluide présente une température qui est supérieure à celle de la zone la plus froide dans le récipient.

3. Procédé conforme à au moins une des revendications 1 et 2,
**caractérisé en ce que**
la température du fluide introduit est choisie de sorte que la surface interne d'un récipient présente après l'égalisation de la température une température située au-dessus du point de rosée de l'agent de stérilisation.
